# EUROPÄISCHE PATENTSCHRIFT

(11) **EP 0 755 399 B1**
(45) Veröffentlichungstag und Bekanntmachung des Hinweises auf die Patenterteilung: **29.09.1999**
(21) Anmeldenummer: 95915838.7
(22) Anmeldetag: 31.03.1995
(51) Int. Cl.: C07F 15/00, A61K 31/28

(54) **LOBAPLATIN-TRIHYDRAT**
LOBAPLATINUM TRIHYDRATE
TRIHYDRATE DE LOBAPLATINE

(30) Priorität: 15.04.1994 DE 4415263
(43) Veröffentlichungstag der Anmeldung: 29.01.1997
(73) Patentinhaber: ASTA Medica Aktiengesellschaft, 01277 Dresden (DE)
(72) Erfinder: GÜNTHER, Eckhard, D-63073 Offenbach (DE); WULF, Jens-Peter, D-63477 Maintal (DE); ENGEL, Jürgen, D-63755 Alzenau (DE); KUTSCHER, Bernhard, D-63477 Maintal (DE)
(74) Vertreter: Decker, Karl-Ludwig
(86) Internationale Anmeldenummer: EP9501214
(87) Internationale Veröffentlichungsnummer: WO9528407

(56) Entgegenhaltungen:
- EP-A- 0 264 109
- EP-A- 0 324 154
- WO-A-90/07514

## Beschreibung

Durch die britische Patentanmeldung 2 024 823 sind beispielsweise Platin-Komplexe der 1,1-Bis(aminomethyl)cyclobutans bekannt. Diese Verbindungen werden zur Behandlung von Krebskrankheiten empfohlen.
Die erfindungsgemäßen Verbindungen besitzen eine gute Antitumorwirksamkeit (beispielsweise in vitro am AH 135-Tumor, B 16-Melanom, Colon 115; in vivo beispielsweise an der P 388 Leukämie der Maus). Darüber hinaus weisen die erfindungsgemäßen Verbindungen nur eine geringe Toxizität auf, insbesondere haben sie keine kumulative Toxizität und besitzen keine Nephrotoxizität.

Im Patent EP 324,154 werden Platinkomplexe des trans-1,2-Bis(aminomethyl)cyclobutan als potente antitumorwirksame Verbindungen beschrieben. Insbesondere hat sich cis-[trans-1,2-Cyclobutanbis(methylamin)-N,N']-[(2S)-lactato-O¹,O²]-platin(II) (INN: Lobaplatin) gegenüber Cisplatin als besser wirksam und besser verträglich erwiesen (R. Voegeli et al. Drugs of the Future 1992, 17, 883-886 und R. Voegeli et al., J. Cancer Res. Clin. Oncol., 1990, 116, 439 - 442).

Die chemische Synthese von Lobaplatin zielte bislang auf die wasserfreie Substanz ab. Wie dem Synthesebeispiel (EP 324 154, Beispiel la) zu entnehmen ist, wurde die wäßrige Reaktionslösung vollständig eingeengt, in Methanol aufgenommen, mit Aktivkohle gerührt, das Filtrat nach Versetzen mit Diethylether und bei Kühlschranktemperatur kristallisiert. Die so gewonnenen Kristalle wurden vakuumgetrocknet. Bei dieser Verfahrensweise gibt es Schwierigkeiten, die Reinheits- und Gehaltsparameter reproduzierbar und innerhalb der vorgeschriebenen Grenzen Schwierigkeiten, die Reinheits- und Gehaltsparameter reproduzierbar und innerhalb der vorgeschriebenen Grenzen einzustellen. Zudem ist die Substanz hygroskopisch und gibt durch Verkleben Schwierigkeiten bei der Dosierung und der galenischen Verarbeitung.

Aufgabe der Erfindung ist es, eine neue Form von Lobaplatin zur Verfügung zu stellen, die einfacher und sicherer zu handhaben ist und die eine verbesserte Qualität aufweist. Es ist weiterhin wichtig, dem Klinikpersonal eine einfach zu handhabende galenische Form zur Verfügung zu stellen, da der Umgang mit Cytostatika aus gewerbehygienischer Sicht nicht unproblematisch ist.
Es wurde nun gefunden, daß die Trihydrat-Form des Lobaplatin in hohem Maße die oben genannten Anforderungen erfüllt.

Hydratformen von Lobaplatin wurden bislang nicht beschrieben. Eine gezielte Untersuchung ergab nun, daß das Trihydrat von Lobaplatin überraschenderweise wesentliche Vorteile aufweist.

Die vorliegende Erfindung beschreibt die Gewinnung von Lobaplatin als Trihydrat und die daraus resultierenden, unerwarteten Vorzüge: einfachere Synthese, Verringerung der Nebenproduktanteile, keine Hydroskopie, reproduzierbar einstellbarer Wassergehalt, verbesserte Lagerstabilität, bessere Dosierbarkeit, bessere pharmazeutische Verarbeitbarkeit. Aus dem Lobaplatin-Trihydrat lassen sich beispielsweise folgende galenische Zubereitungen herstellen: Tabletten, Kapseln, Dragees, Retardformulierungen, Lyophilisate, Injektions- bzw. Infusionslösungen.

Die folgenden Untersuchungen sollen diese Vorteile belegen:

### 1. Synthese

Das bis zum Eintropfen des Dihydroxo-Platinkomplexes in die wäßrige Milchsäurelösung völlig analog dem Beispiel la aus EP 324 154 synthetisierte Lobaplatin wird jetzt wesentlich einfacher aufgearbeitet:

Nach dem Einengen des wäßrigen Reaktionsansatzes kristallisiert rohes Lobaplatin aus und wird unter Zusatz von etwas Aktivkohle aus einem Gemisch aus Wasser / Aceton umkristallisiert. Die so als Trihydrat gewonnenen Kristalle werden an der Luft bei Raumtemperatur oder leicht erhöhter Temperatur getrocknet (siehe Beispiel).

Von 15 Chargen, 7 Chargen als wasserfreie Substanz hergestellt, 8 als Trihydrat, wurde ein analytisches Profil erstellt, daß die deutliche Überlegenheit der als Trihydrat synthetisierten Substanz zeigt.

### 2. Elementaranalyse

| a) wasserfrei: | | |
|---|---|---|
| berechnet | gefunden | Abweichung* |
| C 27,21% | 25,86% | 5,0% |
| H 4,57% | 4,82% | 5,5% |
| N 7,05% | 6,65% | 5,7% |
| Pt49,10% | 46,39% | 6,1% |

| b) Trihydrat: | | |
|---|---|---|
| berechnet | gefunden | Abweichung* |
| C 23,95% | 23,92% | 0,1% |
| H 5,36% | 5,25% | 2,1% |
| N 6,21% | 6,06% | 2,3% |
| Pt43,22% | 43,44% | 0,5% |

| | | |
|---|---|---|
| * Abweichung = 100 % ∗ (berechnet-gefundene / berechnet | | |

### 3. Gehaltsbestimmung

### a) wasserfrei:

Die mittels Karl-Fischer-Titration bestimmten Wassergehaltswerte von 3,73 ± 1,55% zeigen, daß durch die Hygroskopie des Lobaplatins eine vollständige Trocknung nicht möglich ist. Zudem streuen die erreichten Werte sehr stark.

Der mittels HPLC bestimmte Wirkstoffgehalt (unter Abzug der durch Karl-Fischer-Titration bestimmten Wassergehaltswerte) lag mit 98,00 ± 1,38% Wirkstoffgehalt unter den erforderlichen Werten laut Spezifikation und war ebenfalls breit gestreut. Gemäß der Spezifikation muß der Wirkstoffgehalt zwischen 97 % und 102% liegen.

### b) Trihydrat:

Die Wassergehalte der Trihydratchargen lagen mit 12,09 ± 0,09% sehr nahe am theoretischen Wert von 11,96%. Auch die Analysen der Wirkstoffgehaltbestimmung erreichten mit 99,76 ± 0,83% akzeptable Werte, deren Schwankungsbreite innerhalb der in der Pharmazie üblichen Grenzen liegen.

### 4. Verunreinigungsprofil

Mittels Dünnschichtchromatographie wurde der Gehalt für bekannte und unbekannte Verunreinigungen bestimmt. Die Summe der Verunreinigungen wurde für wasserfreie Substanz zu 1,21 ± 0,55% bestimmt, für Trihydrat zu 0,34 ± 0,19%. Auch hier verbesserten sich sowohl Absolutwerte und Streuung.

### 5. Trübung

Verbesserungen wurden auch für die gemessenen Trübungswerte erzielt: lagen diese für wasserfreie Substanz bei 1,45 ± 0,91 FTU, konnten mit Trihydrat 0,38 ± 0,41 FTU erreicht werden.

### 6. Stabilität

Je zwei Chargen wasserfreier Substanz und Trihydrat wurden in Lagerungsversuchen jeweils bei 4°C, Raumtemperatur, 31°C, 41°C und bei 31°C / 80% relative Luftfeuchtigkeit (offene Probenfläschchen) eingelagert. Nach 3, 6 und 12 Monaten wurden das Verunreinigungsprofil (DC), der Wirkstoffgehalt (HPLC), der Wassergehalt (Karl-Fischer-Titration) und die Trübung untersucht. Dabei war für alle Einlagerungsbedingungen festzustellen:
- Der Gehalt an Verunreinigungen steigert sich bei wasserfrei hergestellter Substanz. Beim Trihydrat liegen die Verunreinigungen auf einem konstant niedrigen Niveau.
- Der Wirkstoffgehalt liegt beim Trihydrat eindeutig näher und mit geringerer Streuung am 100%-Wert.
- Die hygroskopische, wasserfreie Substanz zeigt eine Steigerung des Wassergehaltes, während dieser beim erfindungsgemäßen Trihydrat konstant ist.
- Die Trübungen der Lyophilisatchargen, die aus der wasserfreien Substanz hergestellt wurden, beginnen bei deutlich höheren Werten und steigen zudem im Verlaufe der Lagerung wesentlich an. Bei den Trihydratchargen verbleiben die Trübungswerte auf konstant niedrigem Niveau.

Die Herstellung der Lyophilisate erfolgte gemäß der in der DE-OS 38 43 571 angegebenen Vorschrift

### 7. Pharmazeutische Verarbeitbarkeit

Bedingt durch die Hygroskopie der wasserfreien Verbindung muß diese möglichst unter Luftausschluß gehandhabt werden. Es kommt durch hinzutretende Feuchtigkeit zu Verklebungen und Anhaftungen. Das luftstabile Trihydrat zeichnet sich durch gute Rieselfähigkeit und somit deutlich verbesserte Dosier- und Verarbeitbarkeit aus.

### Beispiel für die Herstellung des Lobaplatin-Trihydrats

3,8 g (10 mmol) cis-[trans-1,2-Cyclobutanbis(methylamin)-N,N']-dichloroplatin(II) werden in 20 ml Wasser suspendiert und auf 40 °C erwärmt. 3,39 g (20 mmol) Silbernitrat werden zugegeben. Nach 1,5 stündigem Rühren und nach Abkühlen im Kühlschrank wird vom ausgefallenen Silberchlorid abgesaugt und mit 10 ml Wasser gewaschen. Das Filtrat wird über eine Säule mit 100 ml eines basischen Ionenaustauschers passiert und mit 150 ml Wasser nachgewaschen. Als Ionenaustauscher kann jeder handelsübliche, basische Ionenaustauscher eingesetzt werden. Dabei wird in 4,5 g (10 mmol, 20%ig) L-Milchsäure eingetropft. Nach dreitägigem Rühren wird auf etwa 20 ml eingeengt und über Nacht im Kühlschrank stehengelassen. Die ausgefallenen Kristalle werden abgesaugt, das Filtrat weiter eingeengt und die nach Stehen über Nacht im Kühlschrank ausgefallenen Kristalle werden wiederum abgesaugt. Die vereinigten Kristallmengen werden aus 20 ml Wasser / Aceton (1/1, v/v) umkristallisiert. Ausbeute 2,3 g (51%), Schmelzpunkt 210°C (Zers.).

| C₉H₂₄N₂O₆Pt M=451.38 | | | | |
|---|---|---|---|---|
| berechnet | C 23,95% | H 5,36% | N 6,21% | Pt 43,22% |
| gefunden | C 23,94% | H 5,28% | N 6,15% | Pt 43,05% |
| | C 23,99% | H 5,25% | N 6,05% | |

Wassergehalt (Karl-Fischer-Titration): 12,24% (ber. 11,96%)
¹H-NMR (500 MHz, D₂O): δ (ppm)= 1,2 (d, 3H, CH₃), 1,6 (m, 2H,CH₂-CH₂), 1.9 (m, 2H, CH₂-CH₂), 2.4 (m, 2H, CH₂-NH₂), 2,65 (m, 2H, CH-CH), 2.85 (m, 2H, CH₂-NH₂), 4,05 (q, 1H, CH-CH₃),4,3...5,0 (br, überlagert, NH₂)

## Patentansprüche

1. Cis-[trans-1,2-Cyclobutanbis(methylamin)-N,N']-[(2S)-lactato-O¹,O²]-platin(II)-trihydrat(Lobaplatin-Trihydrat)

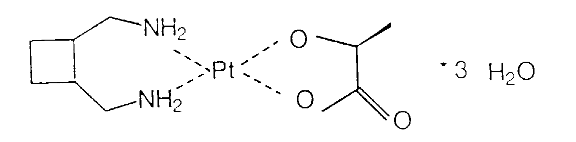

2. Verfahren zur Herstellung von Lobaplatin-Trihydrat, dadurch gekennzeichnet, daß cis-[trans-1,2-Cyclobutanbis(methylamin)-N,N']-dihalogenoplatin (II) durch Austauschreaktionenen der Halogenliganden und anschießende Aufarbeitung in wäßrigem Medium in cis[trans-1,2-Cyclobutanbis(methylamin)-N,N']-[(2S)-lactato-O¹,O²]-platin(II)-trihydrat umgewandelt wird.

3. Arzneimittel, enthaltend cis-[trans-1,2-Cyclobutanbis(methylamin)-N,N']-[(2S)-lactato-O¹,O²]-platin (II)-trihydrat, sowie übliche Träger-, Verdünnung- und/oder Hilfsstoffe.

## Claims

1. Cis-[trans-1,2-cyclobutanebis(methylamine)-N,N']-[(2S)-lactate-O¹,O²)-platinum(II) trihydrate (lobaplatin trihydrate)

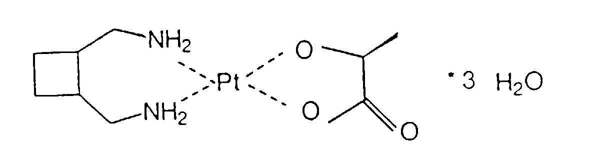

2. Process for the preparation of lobaplatin trihydrate, characterized in that cis-[trans-1,2-cyclobutanebis(methylamine)-N,N']-dihalogenoplatinum(II) is converted into cis-[trans-1,2-cyclobutanebis(methylamine)-N,N']-[(2S)-lactate-O¹,O²)-platinum(II) trihydrate by exchange reactions of the halogen ligands and subsequent working up in an aqueous medium.

3. Medicament comprising cis-[trans-1,2-cyclobutanebis(methylamine)-N,N']-[(2S)-lactate-O¹,O²]-platinum(II) trihydrate and customary excipients, diluents and/or auxiliaries.

## Revendications

1. Cis-[trans-1,2-Cyclobutanbis(méthylamino)-N,N']-[(2S)-lactato-O¹, O²]-platine (II)-trihydrate (trihydrate de Lobaplatine)

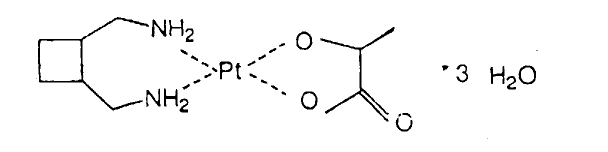

2. Procédé de préparation du trihydrate de Lobaplatine,
caractérisé en ce qu'
on convertit le Cis-[trans-1,2-Cyclobutanbis(méthylamino)-N,N']-dihalogénoplatine (II) par des réactions d'échange des ligands halogène et traitement subséquent en milieu aqueux en trihydrate de cis-[trans-1,2-Cyclobutanbis(méthylamino)-N,N']-[(2S)-lactato-O¹, O²]-platine.

3. Médicament contenant du trihydrate de cis-[trans-1,2-Cyclobutanbis(méthylamino)-N,N']-[(2s)-lactato-O¹, O²]-platine (II), ainsi que des supports, des agents de dilution, et/ou des adjuvants.
